# FASCICULE DE BREVET EUROPEEN

(11) **EP 2 977 078 B1**
(45) Date de publication et mention de la délivrance du brevet: **25.10.2017**
(21) Numéro de dépôt: 15176474.3
(22) Date de dépôt: 13.07.2015
(51) Int. Cl.: A61N 1/36

(54) **DISPOSITIF MÉDICAL IMPLANTABLE ACTIF DE THÉRAPIE PAR STIMULATION DU NERF VAGUE, AVEC AJUSTEMENT DYNAMIQUE DES PÉRIODES DE STIMULATION**
AKTIVE IMPLANTIERBARE MEDIZINISCHE VORRICHTUNG ZUR STIMULATION DES VAGUSNERVS MIT DYNAMISCHER ANPASSUNG DER STIMULATIONSPERIODEN
ACTIVE IMPLANTABLE MEDICAL DEVICE FOR THERAPY BY VAGUS NERVE STIMULATION, WITH DYNAMICALLY ADJUSTING STIMULATION PERIODS

(30) Priorité: 23.07.2014 FR 1457109
(43) Date de publication de la demande: 27.01.2016
(73) Titulaire: Sorin CRM SAS, 92140 Clamart Cedex (FR); Université de Rennes 1, 35065 Rennes Cedex (FR); INSERM - Institut National de la Santé et de la Recherche Médicale, 75013 Paris (FR)
(72) Inventeur: Bonnet, Jean-Luc, 91300 Massy (FR)
(74) Mandataire: Grünecker Patent- und Rechtsanwälte PartG mbB

(56) Documents cités:
- WO-A1-2014/074523
- US-A1- 2006 122 675
- US-A1- 2007 255 374
- US-A1- 2012 172 741

## Description

L'invention concerne les "dispositifs médicaux implantables actifs" tels que définis par la directive 90/385/CEE du 20 juin 1990 du Conseil des communautés européennes.

Elle concerne plus précisément les implants permettant de délivrer des thérapies de stimulation du système nerveux, notamment de stimulation du nerf vague. Ce type de stimulation sera ici désigné "neurostimulation" ou "stimulation VNS" (*Vagus Nerve Stimulation*). Le dispositif comprend à cet effet une sonde pourvue d'une électrode implantée sur le nerf vague et un générateur délivrant des impulsions VNS sur cette électrode.

La stimulation du système nerveux est une thérapie reconnue à l'égard de très nombreux troubles tels que l'épilepsie, la douleur, l'insuffisance cardiaque, l'apnée, l'obésité, etc. Pour le traitement des troubles tels que l'insuffisance cardiaque, l'épilepsie ou l'obésité, les dispositifs utilisés comprennent typiquement une sonde pourvue d'une électrode implantée sur le nerf vague (dite "sonde VNS") et un générateur délivrant des impulsions VNS sur cette électrode.

Dans certaines thérapies, le profil de stimulation VNS est composé de salves ou trains d'impulsions répétitives produites pendant des périodes dites "d'activité" ou "périodes ON" de quelques dizaines de secondes, entrecoupées par des périodes dites "d'inactivité" ou "périodes OFF" de quelques minutes durant lesquelles la stimulation n'est plus délivrée.

Le nerf vague peut être éventuellement stimulé de façon synchrone avec le rythme cardiaque, auquel cas le dispositif comprend des moyens de recueil des ondes de dépolarisation du myocarde, typiquement des moyens de recueil d'un ECG par une électrode subcutanée, ou d'un EGM par une électrode implantée sur ou dans le myocarde.

La stimulation VNS est en particulier bien adaptée au traitement des troubles cardiaques, notamment chez les patients en risque d'insuffisance cardiaque, où la stimulation du nerf vague agit sur les fonctions cardio-vasculaires par réduction du rythme cardiaque, réduction de la contractilité du myocarde et augmentation de la durée de la diastole, ce qui peut aider à réduire l'évolution d'un remodelage cardiaque susceptible de mener à un état d'insuffisance cardiaque aggravée.

En effet, chez un patient souffrant d'insuffisance cardiaque, ou dans le post-infarctus du myocarde, l'activité du système sympathique est excessive (état sympathique hypertonique), avec au contraire un système parasympathique déprimé, ce qui conduit à un rythme cardiaque plus rapide que la normale.

Le problème abordé par l'invention est lié au fait qu'une thérapie neuronale par stimulation VNS, si elle se révèle efficace au début de son application, voit cette efficacité diminuer rapidement, vraisemblablement du fait de phénomènes de compensation provenant de la formation d'une boucle de contrôle physiologique.

Ainsi, si l'on mesure par exemple la fréquence cardiaque (ou l'intervalle RR) du patient juste avant et juste après le déclenchement de la stimulation VNS (c'est-à-dire juste avant et juste après la transition de OFF vers ON), on constate un ralentissement important du rythme cardiaque, qui atteint un maximum après une dizaine de secondes environ. Mais ensuite, toutes choses égales par ailleurs, la fréquence recommence à augmenter progressivement alors même que la stimulation VNS continue à être appliquée. Au bout de quelques dizaines de secondes, le ralentissement du rythme cardiaque procuré par la stimulation VNS n'est plus que de 80 % à 60 % de ce qu'il était initialement, c'est-à-dire à l'instant où la stimulation VNS avait commencé à être appliquée. Toutefois, si la stimulation VNS est arrêtée (transition de ON vers OFF) puis réactivée plus tard (fin de la période OFF), l'efficacité initiale est retrouvée, avec ensuite le même affaiblissement progressif de l'effet de la thérapie.

Il n'y a donc aucun avantage (au contraire) à appliquer de façon ininterrompue une stimulation VNS, et c'est pour cette raison que la technique d'alternance de périodes ON et de périodes OFF est mise en oeuvre.

Un autre aspect dont il y a lieu de tenir compte est celui des phénomènes délétères tels que toux, apnées, contractions ventriculaires ectopiques ou PVCs (*Premature Ventricular Contractions*), qui peuvent apparaitre en tant qu'effets secondaires de la stimulation VNS : si de tels symptômes se présentent, il convient de réduire la stimulation VNS afin que les inconvénients de celle-ci ne l'emportent pas sur les bénéfices.

Aujourd'hui, les durées des périodes ON et OFF sont programmées de manière essentiellement empirique par le praticien. Celui-ci doit trouver un compromis entre une période ON suffisamment longue pour que la stimulation VNS soit bénéfique au patient, tout en évitant qu'une stimulation prolongée ne produise des effets délétères tels que survenue de toux, etc. Concrètement, le praticien doit suivre les patients sur une longue durée de manière à ajuster finement les durées des périodes ON et OFF au mieux de chaque patient.

Il n'a été présenté jusqu'à présent aucun exposé raisonné de raisons et de procédures qui pourraient aider les praticiens à programmer ces paramètres, en particulier dans le domaine de la gestion du rythme cardiaque - notamment pour des patients en situation d'insuffisance cardiaque - où, comme on l'a exposé plus haut, la durée des périodes ON a une incidence notable sur les variations des paramètres rythmiques et/ou hémodynamiques.

Le but de l'invention est de proposer un dispositif permettant de remédier aux inconvénients ci-dessus par un ajustement automatique, dynamique, des périodes ON permettant de maximiser le bénéfice tiré par le patient de la stimulation VNS et, d'autre part, évitant l'apparition de phénomènes délétères susceptibles d'être induits par cette stimulation VNS.

Le WO 2007/127150 A1 (EP 2 019 714 A1) propose, pour éviter les phénomènes de compensation, de modifier le "protocole thérapeutique" VNS par modulation des durées ON/OFF appliquées (en gardant le même rapport cyclique) soit de façon périodique au bout d'une durée prédéterminée, soit sur détection d'un événement tel qu'une activation externe par l'utilisateur ou le praticien, ou bien un signal délivré par un capteur. Les protocoles eux-mêmes, notamment les durées des périodes ON, sont toutefois déterminés *a priori,* de manière arbitraire et sans relation avec un paramètre physiologique qui reflèterait l'état courant du patient à un instant donné.

Le US 2006/0015153 A1 (US 7 483 747 B2) propose de recalculer de temps à autres les durées des périodes ON et OFF, à intervalles réguliers ou non, ou aléatoirement. Mais, ici encore, cette technique ne prend pas en compte les effets instantanés de la thérapie VNS.

Le US 2012/172741 A1 propose de recalculer la durée des périodes ON pour tenir compte des modifications physiologiques à dynamique lente, telles que variations d'impédance, fibrose ou modification des tissus nerveux, susceptibles de modifier sur le long terme la réponse physiologique à la thérapie VNS. La méthode proposée met en oeuvre une boucle fermée (*closed loop*) qui adapte en continu le rapport cyclique, mais sans considération de seuil ni des durées absolues des périodes ON et OFF.

Le WO 2014/074523 A1 décrit encore un autre système de thérapie VNS, opérant selon un principe de modification d'une thérapie "de maintien". Cette approche consiste à définir un niveau de rapport cyclique ON-OFF de maintien et à l'adapter, mais seulement pendant les périodes où un événement physiologique prédéfini (par exemple une tachyarythmie) est détecté. L'adaptation se réalise en augmentant itérativement la valeur de maintien de rapport cyclique, sans pour autant atteindre un certain niveau maximal qui pourrait produire des effets indésirables.

L'idée de base de l'invention consiste, au contraire, à adapter quasiment en temps réel, et en continu (et non pas uniquement lors de la détection d'un événement particulier, comme dans le cas du WO 2014/074523 A1 précité), la durée à la fois des périodes ON et OFF à chaque cycle de stimulation VNS en fonction d'un paramètre physiologique mesuré en continu, représentatif de l'activité cardiaque et/ou de l'état hémodynamique du patient. Ce paramètre, qui fournit un indicateur direct de l'efficacité de la stimulation VNS sur les fonctions qui font l'objet de la thérapie, permet de piloter directement l'application des impulsions VNS afin d'en maximiser le bénéfice pour le patient.

Plus précisément, l'invention propose un dispositif implantable de thérapie neuronale VNS par stimulation du nerf vague ou de l'une de ses branches comprenant, de manière en elle-même connue notamment d'après le US 2012/172741 A1 précité : un générateur apte à produire des séquences d'impulsions de stimulation VNS générées continument en succession pendant des périodes d'activité séparées par des périodes intercalaires d'inactivité durant lesquelles aucune stimulation n'est délivrée ; des moyens d'analyse recevant en entrée un signal, délivré par un capteur physiologique, représentatif de l'activité cardiaque et/ou de l'état hémodynamique du patient porteur du dispositif, et délivrant en sortie du générateur un paramètre témoin de l'efficacité courante de la thérapie VNS ; et des moyens de contrôle dynamique de la thérapie VNS, aptes à moduler la durée des périodes d'activité en fonction de la valeur courante du paramètre témoin délivrée par les moyens d'analyse.

De façon caractéristique de l'invention, les moyens de contrôle dynamique de la thérapie VNS sont aptes à moduler à chaque cycle de stimulation VNS la durée de la période d'activité, et sont en outre aptes à calculer, à la fin de chaque période d'activité, la durée de la période d'inactivité en fonction de la durée de la période d'activité précédente.

Selon diverses caractéristiques subsidiaires avantageuses :
- les moyens de contrôle dynamique de la thérapie VNS sont aptes à moduler la durée des périodes d'activité par comparaison du paramètre témoin avec un seuil prédéterminé ;
- les moyens de contrôle dynamique de la thérapie VNS sont aptes à moduler la durée des périodes d'inactivité de manière à maintenir constant le rapport cyclique entre périodes d'activité et périodes d'inactivité ;
- les moyens de contrôle dynamique de la thérapie VNS comprennent des moyens aptes à surveiller, de préférence à chaque cycle cardiaque, le franchissement d'une valeur de seuil par la valeur courante du paramètre témoin, et des moyens aptes à mettre fin, de préférence à chaque cycle cardiaque, à la période d'activité à partir du franchissement de ce seuil ;
- ce seuil peut être un seuil prédéterminé fixe, ou bien un seuil dynamique, le dispositif comprenant alors en outre des moyens de calcul d'un seuil du paramètre témoin pour chaque période d'activité courante ;
- dans ce dernier cas, les moyens de calcul du seuil peuvent comprendre des moyens de calcul du seuil en fonction d'une valeur d'extremum du paramètre témoin atteinte consécutivement au déclenchement de la période d'activité courante ;
- les moyens de calcul du seuil sont alors avantageusement des moyens aptes à calculer ce seuil en fonction : de l'écart mesuré entre i) une valeur de base du paramètre témoin avant le déclenchement de la période d'activité courante et ii) la valeur d'extremum du paramètre témoin atteinte après le déclenchement de cette période d'activité courante ; d'une valeur de base du paramètre témoin avant le déclenchement de la période d'activité courante ; et/ou de la valeur d'extremum du paramètre témoin atteinte après le déclenchement de cette période d'activité courante ;
- le dispositif comprend en outre des moyens de détection de la survenue de phénomènes délétères, tels que toux, extrasystoles ventriculaires et/ou apnées, et des moyens aptes à inhiber le déclenchement d'une séquence d'impulsions VNS par le générateur en cas de survenue d'un événement délétère ; il est alors avantageusement prévu des moyens temporisateurs pour mettre fin inconditionnellement à la génération de la séquence d'impulsions VNS après écoulement d'un délai prédéterminé.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une représentation schématique des différents éléments mis en oeuvre par le dispositif de l'invention, placés dans leur contexte.
La Figure 2 illustre sous forme de schéma par blocs les différentes fonctions mises en oeuvre par le dispositif de l'invention.
La Figure 3 illustre l'effet de l'application d'une salve d'impulsions VNS sur le rythme cardiaque, effet reflété ici par la variation de l'intervalle RR.
La Figure 4 est homologue de la Figure 3, montrant l'effet de la stimulation VNS suite à la mise en oeuvre de l'invention.
Les Figures 5a et 5b sont des chronogrammes relevés pour deux patients différents lors d'une étude clinique, montrant les variations du rythme cardiaque reflétées par la variation de l'intervalle RR suite à l'application d'une thérapie VNS selon les enseignements de l'invention.
La Figure 6 illustre, pour trois cycles de stimulation VNS, les variations liées entre elles des durées des périodes ON et OFF.
La Figure 7 illustre l'alternance des durées, variables, des périodes ON et OFF au cours du temps.
La Figure 8 est un organigramme général présentant l'enchainement des différentes étapes de mise en oeuvre d'un dispositif de stimulation VNS selon l'invention.

On va maintenant décrire un exemple de réalisation de l'invention.

En ce qui concerne ses aspects logiciels, l'invention peut être mise en oeuvre notamment par une programmation appropriée du logiciel de commande d'un stimulateur connu de stimulation du nerf vague (stimulateur VNS).

Un tel stimulateur comprend un microprocesseur programmable pourvu de circuits pour mettre en forme et délivrer des impulsions de stimulation à des électrodes implantées. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs correspondant en pratique à une pluralité de fonctions globalement exécutées par un même logiciel.

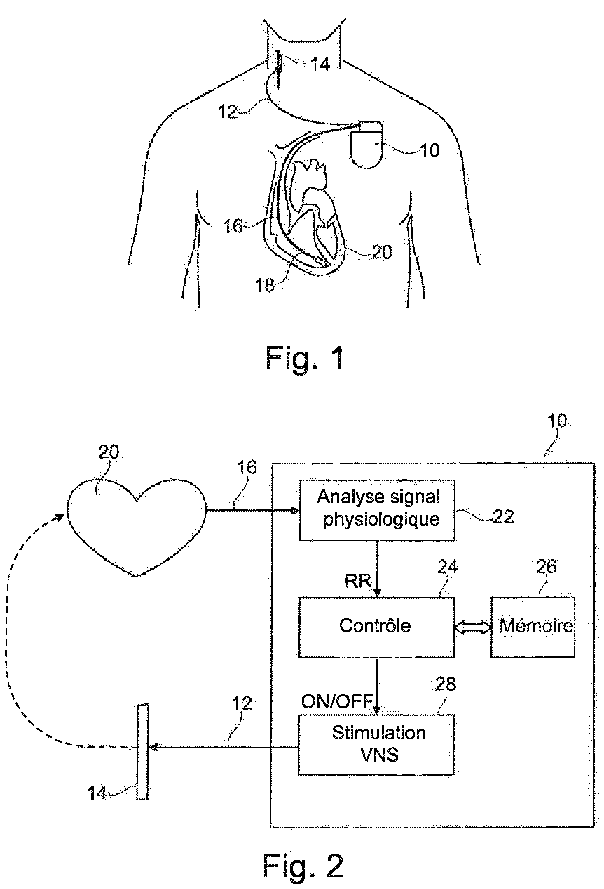

Sur la Figure 1, on a illustré un ensemble comprenant un générateur VNS implantable 10 assurant la production d'impulsions de stimulation transmises par une sonde 12 à une électrode appliquée sur le nerf vague 14 ou disposée à proximité de celui-ci ou de l'une de ses branches. Le générateur 10 est également pourvu d'une sonde cardiaque 16 munie à son extrémité distale 18 d'une électrode de recueil de l'activité électrique du myocarde 20. Cette sonde 16 recueille des signaux d'électrogramme endocavitaire (EGM) qui permettront de délivrer un signal représentatif de l'activité cardiaque du patient, en l'occurrence la fréquence cardiaque, déterminée par la durée des intervalles RR des dépolarisations cardiaques successives.

On notera que cette manière d'obtenir un signal représentatif de l'activité cardiaque du patient n'est pas limitative, et qu'il est possible d'utiliser d'autres signaux pour la mise en oeuvre de l'invention, en variante ou en complément. On peut notamment utiliser des signaux d'accélération endocardiaque (EA), qui permettent d'obtenir un paramètre représentatif de l'état hémodynamique du patient, par exemple un des paramètres décrits dans le EP 2 092 885 A1 (ELA Médical) tels que l'amplitude crête-à-crête du premier pic d'accélération endocardiaque (PEA1), l'intervalle entre le premier et le second pic EA, etc.

De façon générale, le signal recueilli doit permettre de fournir au générateur un paramètre témoin de l'efficacité instantanée de la thérapie VNS, ce paramètre témoin pouvant être dérivé des signaux délivrés par d'autres types de capteurs physiologiques que ceux illustrés dans cet exemple, par exemple un capteur de pression artérielle, un capteur de ventilation-minute, etc. Le choix de l'intervalle RR comme paramètre témoin dans la suite de la description ne doit donc être considéré comme en aucune manière limitatif de l'invention.

La Figure 2 illustre sous forme de schéma par blocs les différentes fonctions mises en oeuvre par le dispositif de l'invention.

Le signal cardiaque (signal EGM, signal EA, ...) recueilli par la sonde 16 est analysé (bloc 22) de manière à délivrer un paramètre témoin de l'efficacité instantanée de la thérapie VNS, ce paramètre étant dans le présent exemple le rythme cardiaque, reflété par la valeur des intervalles RR successifs.

Ce paramètre témoin est appliqué à une unité de contrôle (bloc 24) reliée à une mémoire 26 où sont conservés divers réglages et seuils, et pouvant conserver un historique des déclenchements et des arrêts des phases de stimulation VNS. L'unité de contrôle 24 pilote un générateur 28 d'impulsions, sélectivement pendant des périodes d'activité (périodes ON) séparées par des périodes intercalaires d'inactivité (périodes OFF) durant lesquelles aucune stimulation électrique n'est délivrée au nerf vague 14 via la sonde 12.

Une stimulation VNS produit sur l'activité cardiaque un certain nombre d'effets, qui peuvent être de nature :
- chronotrope : diminution du rythme cardiaque, c'est-à-dire augmentation des intervalles RR ;
- dromotrope : diminution de la vitesse de conduction AV, conduisant à une augmentation des intervalles PR ;
- bathmotrope : diminution de l'excitabilité des myosites ;
- inotrope : réduction de la contractilité cardiaque ; et/ou
- lusitrope : augmentation de la vitesse de relaxation cardiaque.

La stimulation VNS a également un effet sur le système vasculaire par modulation de la vasoconstriction, avec une modification des diamètres des artères et de la résistance périphérique se traduisant par une vasodilatation générale du système vasculaire.

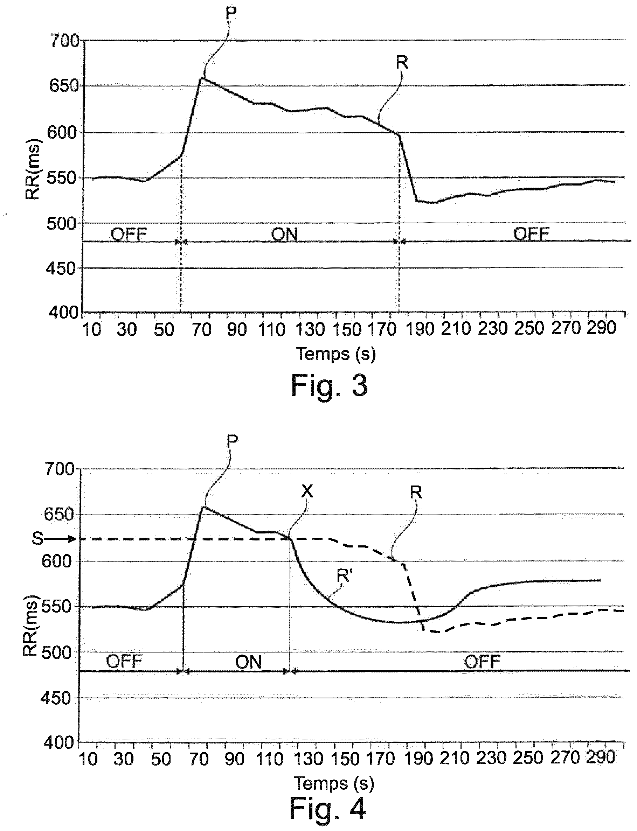

En référence à la Figure 3, qui représente la variation de l'intervalle RR au cours d'une alternance de périodes OFF/ON/OFF chez un patient (toutes choses égales par ailleurs), on constate que la réponse chronotrope à une stimulation VNS, reflétée par les variations du rythme cardiaque (intervalles RR) se traduit par quatre phases successives lorsque la stimulation VNS est appliquée, c'est-à-dire à la suite d'une transition d'une période OFF à une période ON :
- augmentation rapide de l'intervalle RR, jusqu'à un pic P ;
- après le pic P, phase de pseudo-plateau avec réduction progressive de l'intervalle RR (région repérée R sur la Figure 3) ;
- à l'arrêt de la stimulation VNS (c'est-à-dire à la transition de ON vers OFF), diminution rapide de l'intervalle RR, c'est-à-dire accélération du rythme cardiaque ;
- enfin, phase de récupération ou de "rebond", qui reflète les réponses transitoires baroréflexes conduisant à une stabilisation finale de la pression sanguine et du rythme cardiaque à la valeur qu'ils avaient avant l'application de la stimulation VNS.

Ces quatre phases peuvent être également observées sur la réponse ino-tropique (variation de contractilité cardiaque) ainsi que sur les autres types de réponses, éventuellement avec une dynamique plus lente due à des constantes de temps plus importantes. C'est pour cette raison que l'on choisit de préférence - mais de façon non limitative - l'analyse du rythme cardiaque, reflété en particulier par la variation de l'intervalle RR, comme paramètre témoin de l'efficacité instantanée de la thérapie VNS.

Comme illustré Figure 4, de façon caractéristique de l'invention, le déclenchement de la période OFF est contrôlé à chaque cycle de stimulation VNS de façon à réduire la durée de la période ON lorsque l'effet de la stimulation VNS descend au-dessous d'un seuil prédéterminé.

À cet effet, la valeur courante de l'intervalle RR est comparée à un seuil S, et lorsque ce seuil est franchi (point X sur la Figure 4) la stimulation VNS est arrêtée, c'est-à-dire que l'on déclenche une transition d'une période ON vers une période OFF. L'intervalle RR diminue alors rapidement (courbe R'), beaucoup plus vite que dans le cas précédent de la Figure 3 (illustré en tiretés en R sur la Figure 4).

Cette technique permet d'adapter la stimulation VNS de façon dynamique d'un patient à l'autre, et d'un moment de la journée à un autre pour un même patient, de façon à éviter d'appliquer trop longtemps une thérapie produisant trop peu d'effets positifs pour le patient. De façon optimale, la transition de la période ON vers la période OFF est testée et éventuellement déclenchée à chaque cycle de stimulation VNS.

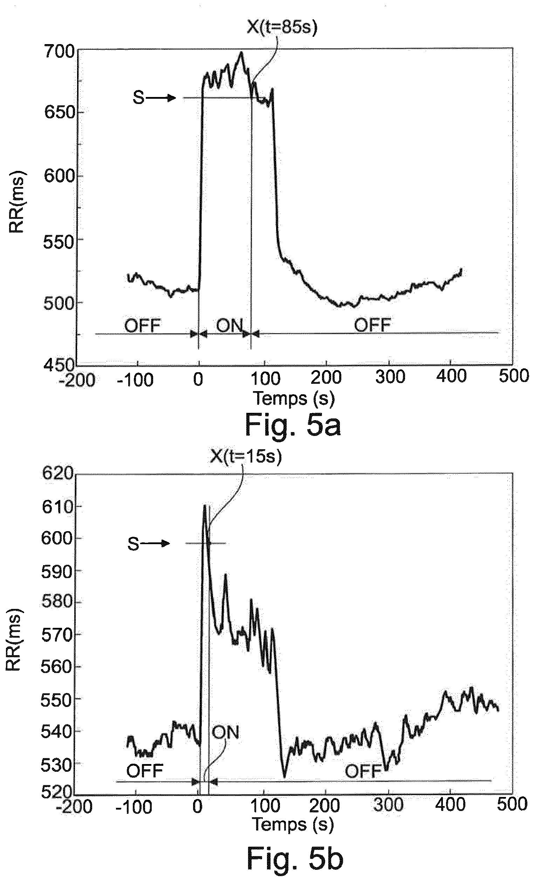

On a représenté Figures 5a et 5b pour deux patients différents les variations de l'intervalle RR relevées dans le cadre d'une étude clinique.

On voit que pour le patient de la Figure 5a la stimulation VNS produit des effets positifs pendant une durée relativement longue à partir du moment où elle est appliquée, le seuil S n'étant franchi qu'après t = 85 secondes environ. En revanche, pour le patient de la Figure 5b, l'efficacité de la stimulation décroit très vite après que le pic a été atteint, de sorte qu'il n'est pas nécessaire de prolonger trop longtemps la thérapie : dans ce cas, le seuil S est franchi après t = 15 secondes seulement et la stimulation VNS n'est pas prolongée au-delà.

Dans une première forme de mise en oeuvre, le seuil S est un seuil fixe par rapport à la valeur de l'intervalle RR atteinte au pic P, par exemple un seuil S fixé à 20 millisecondes au-dessous du niveau du pic P.

Dans une autre forme de mise en oeuvre, l'intervalle entre le seuil et la valeur de pic est variable, par exemple défini par un pourcentage de l'écart entre la valeur de base de l'intervalle RR (valeur moyenne calculée sur un certain nombre de cycles pendant la période OFF juste avant la transition vers la période ON) et la valeur de l'intervalle RR correspondant au pic atteint après la transition de la période OFF vers la période ON. Le seuil peut être par exemple calculé comme étant égal à 25 % de la différence entre la valeur RR de base et la valeur RR de pic : dans l'exemple illustré Figure 4, si la valeur de pic est de 660 ms et la valeur de base 550 ms, le seuil sera défini par : (660-550) x 0,25 = 27,5 ms au-dessous de la valeur de pic.

Dans une autre forme encore de mise en oeuvre, le seuil S peut être calculé à partir de la valeur RR de base seule, ou bien de la valeur RR de pic seule.

Avantageusement, le procédé de l'invention, après avoir mis fin dynamiquement à la stimulation VNS, adapte la durée de la période OFF qui suit en fonction de la durée de la période ON qui vient juste d'être commandée.

Le calcul de la période OFF se fait avantageusement en choisissant de conserver un rapport cyclique (*duty cycle*) constant, c'est-à-dire un ratio constant entre la durée des périodes ON et celle des périodes ON+OFF. Par exemple, si le rapport cyclique est fixé à 1:4, la durée de la période OFF sera de trois fois la durée de la période ON qui la précède immédiatement.

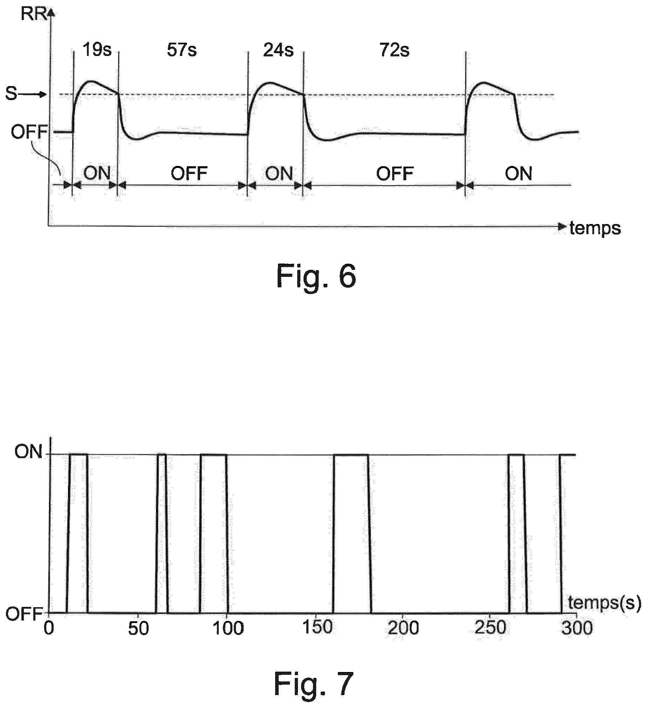

Sur la Figure 6 on a illustré un exemple de trois périodes ON consécutives séparées par des périodes OFF. La première période ON a été interrompue au bout de 19 s, et la durée de la période OFF suivante sera donc calculée à la fin de la période ON en fonction de la durée de cette période, soit 19 x 3 = 57 s. Pour la période ON suivante qui sera interrompue au bout de 24 s, la durée de la période OFF consécutive sera de 24 x 3 = 72 s, et ainsi de suite. Le rapport cyclique ON:OFF peut être choisi parmi toute valeur de l'intervalle ]0 %, 100 %[.

Comme illustré Figure 7, on pourra ainsi observer au cours du temps une succession de périodes ON de durée variable, elles-mêmes suivies de périodes OFF intercalaires de durée également variable, mais avec un rapport cyclique maintenu constant.

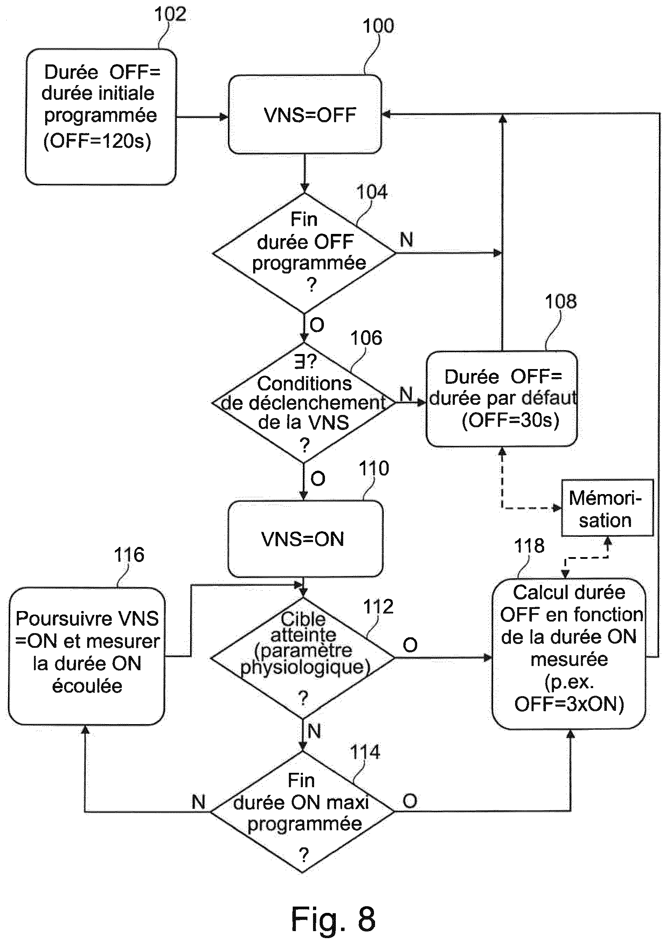

La Figure 8 est un organigramme montrant le déroulement des différentes étapes du procédé de l'invention.

Initialement, le dispositif se trouve dans une configuration où aucune stimulation VNS n'est appliquée (VNS = OFF, bloc 100), la durée OFF étant ajustée à une durée initiale prédéterminée (bloc 102) ; cette valeur peut être soit une valeur programmée par le médecin, par exemple de 120 secondes, soit une valeur calculée par le dispositif en fonction de l'historique du patient.

À la fin de la période initiale (bloc 104), le dispositif vérifie (bloc 106) si les conditions sont réunies pour autoriser le déclenchement d'une stimulation VNS. Le dispositif contrôle en particulier l'absence de toux ou de phénomènes d'apnée (par exemple par une analyse du signal de ventilation minute), l'absence de contractions ventriculaires ectopiques (par une analyse du signal EGM), etc.

Si l'un de ces phénomènes est présent, la période OFF est maintenue et réactivée pour une durée prédéterminée, par exemple de 30 secondes (bloc 108).

En l'absence de phénomène empêchant la délivrance d'une thérapie VNS, la stimulation est appliquée (VNS = ON, bloc 110) et le paramètre physiologique témoin, par exemple l'intervalle RR, est surveillé en permanence.

Si la cible prédéfinie est atteinte, par exemple si l'intervalle RR retombe au-dessous d'un seuil prédéterminé (bloc 112), alors la durée de la période OFF consécutive est calculée de manière à conserver un rapport cyclique constant (par exemple OFF = 3 x ON) (bloc 118) et il est mis fin à la stimulation VNS (transition vers une période OFF, retour au bloc 100).

Dans le cas contraire, si une durée maximale préprogrammée est atteinte (bloc 114) alors de la même manière la durée de la période OFF qui va suivre est calculée et la stimulation VNS est arrêtée (blocs 118, 100). Si la durée préprogrammée n'est pas encore atteinte, la stimulation VNS est poursuivie (bloc 116) et le processus retourne au bloc 112 de surveillance du franchissement du seuil.

## Revendications

1. Un dispositif médical implantable actif de thérapie neuronale VNS par stimulation du nerf vague ou de l'une de ses branches, ce dispositif comprenant :
- un générateur (10), apte à produire des séquences d'impulsions de stimulation VNS générées continument en succession pendant des périodes d'activité (ON) séparées par des périodes intercalaires d'inactivité (OFF) durant lesquelles aucune stimulation n'est délivrée ;
- des moyens d'analyse (22) recevant en entrée un signal, délivré par un capteur physiologique, représentatif de l'activité cardiaque et/ou de l'état hémodynamique du patient porteur du dispositif, et délivrant en sortie du générateur un paramètre témoin de l'efficacité courante de la thérapie VNS ; et
- des moyens (24) de contrôle dynamique de la thérapie VNS, aptes à moduler la durée des périodes d'activité en fonction de la valeur courante du paramètre témoin délivrée par les moyens d'analyse, **caractérisé en ce que** :
· les moyens de contrôle dynamique de la thérapie VNS sont aptes à moduler à chaque cycle de stimulation VNS la durée de la période d'activité (ON),
· et sont en outre aptes à calculer, à la fin de chaque période d'activité (ON), la durée de la période d'inactivité (OFF) en fonction de la durée de la période d'activité (ON) précédente,
et **en ce que** les moyens de contrôle dynamique de la thérapie VNS comprennent :
- des moyens aptes à surveiller le franchissement d'une valeur de seuil (S) par la valeur courante du paramètre témoin (RR) ; et
- des moyens aptes à mettre fin à la période d'activité à partir du franchissement de ce seuil.

2. Le dispositif de la revendication 1, dans lequel les moyens de contrôle dynamique de la thérapie VNS sont aptes à moduler la durée des périodes d'activité (ON) par comparaison du paramètre témoin avec un seuil prédéterminé.

3. Le dispositif de la revendication 1, dans lequel les moyens de contrôle dynamique de la thérapie VNS sont aptes à moduler la durée des périodes d'inactivité (OFF) de manière à maintenir constant le rapport cyclique entre périodes d'activité (ON) et périodes d'inactivité (OFF).

4. Le dispositif de la revendication 1, dans lequel lesdits moyens de contrôle dynamique de la thérapie VNS comprennent :
- des moyens aptes à surveiller à chaque cycle cardiaque le franchissement de ladite valeur de seuil (S) par la valeur courante du paramètre témoin (RR) ; et
- des moyens aptes à mettre fin à chaque cycle cardiaque à la période d'activité à partir du franchissement de ce seuil.

5. Le dispositif de la revendication 1, dans lequel le seuil (S) est un seuil prédéterminé fixe.

6. Le dispositif de la revendication 1, dans lequel le seuil (S) est un seuil dynamique, et le dispositif comprend en outre des moyens de calcul d'un seuil du paramètre témoin pour chaque période d'activité courante.

7. Le dispositif de la revendication 6, dans lequel les moyens de calcul du seuil comprennent des moyens de calcul du seuil en fonction d'une valeur d'extremum (P) du paramètre témoin atteinte consécutivement au déclenchement de la période d'activité courante.

8. Le dispositif de la revendication 7, dans lequel les moyens de calcul du seuil sont aptes à calculer le seuil en fonction de l'écart mesuré entre i) une valeur de base du paramètre témoin avant le déclenchement de la période d'activité courante et ii) la valeur d'extremum du paramètre témoin atteinte après le déclenchement de cette période d'activité courante.

9. Le dispositif de la revendication 7, dans lequel les moyens de calcul du seuil sont aptes à calculer le seuil en fonction d'une valeur de base du paramètre témoin avant le déclenchement de la période d'activité courante.

10. Le dispositif de la revendication 9, dans lequel les moyens de calcul du seuil sont aptes à calculer le seuil en fonction de la valeur d'extremum du paramètre témoin atteinte après le déclenchement de cette période d'activité courante.

11. Le dispositif de la revendication 1, comprenant en outre des moyens de détection de la survenue de phénomènes délétères, et des moyens aptes à inhiber le déclenchement d'une séquence d'impulsions VNS par le générateur en cas de survenue d'un événement délétère.

12. Le dispositif de la revendication 11, dans lequel les phénomènes délétères sont de phénomènes du groupe comprenant : toux, extrasystoles ventriculaires, apnées.

13. Le dispositif de la revendication 11, comprenant en outre des moyens temporisateurs, aptes à mettre fin inconditionnellement à la génération de la séquence d'impulsions VNS après écoulement d'un délai prédéterminé.

## Patentansprüche

1. Aktive implantierbare medizinische Vorrichtung zur neuronalen VNS Therapie durch Stimulation des Vagusnervs oder eines seiner Äste, wobei diese Vorrichtung Folgendes umfasst:
- einen Generator (10), der dazu geeignet ist, kontinuierlich nacheinander generierte Sequenzen von VNS Stimulationsimpulsen während Aktivitätsperioden (ON) zu erzeugen, die durch dazwischenliegende Inaktivitätsperioden (OFF) getrennt sind, während denen keine Stimulation ausgegeben wird;
- Analysemittel (22), die ein durch einen physiologischen Sensor ausgegebenes Eingangssignal empfangen, das die Herzaktivität und/oder den hämodynamischen Zustand des die Vorrichtung tragenden Patienten repräsentiert, und die über den Generator einen Kontrollparameter des aktuellen Wirkungsgrads der VNS Therapie ausgibt; und
- Mittel (24) zur dynamischen Steuerung der VNS Therapie, die dazu geeignet sind, die Dauer der Aktivitätsperioden in Abhängigkeit des aktuellen Werts des durch die Analysemittel ausgegebenen Kontrollparameters zu modulieren, **dadurch gekennzeichnet, dass**:
• die Mittel zur dynamischen Steuerung der VNS Therapie dazu geeignet sind, in jedem VNS Stimulationszyklus die Dauer der Aktivitätsperiode (ON) zu modulieren,
• und ferner dazu geeignet sind, am Ende jeder Aktivitätsperiode (ON), die Dauer der Inaktivitätsperiode (OFF) in Abhängigkeit der Dauer der vorhergehenden Aktivitätsperiode (ON) zu berechnen,
und dass die Mittel zur dynamischen Steuerung der VNS Therapie Folgendes umfassen:
- Mittel, die dazu geeignet sind, die Überschreitung eines Grenzwerts (S) durch den aktuellen Wert des Kontrollparameters (RR) zu überwachen; und
- Mittel, die dazu geeignet sind, die Aktivitätsperiode bei Überschreitung dieses Grenzwerts zu beenden.

2. Vorrichtung nach Anspruch 1, wobei die Mittel zur dynamischen Steuerung der VNS Therapie dazu geeignet sind, die Dauer der Aktivitätsperioden (ON) durch einen Vergleich des Kontrollparameters mit einem vorbestimmten Grenzwert zu modulieren.

3. Vorrichtung nach Anspruch 1, wobei die Mittel zur dynamischen Steuerung der VNS Therapie dazu geeignet sind, die Dauer der Inaktivitätsperioden (OFF) derart zu modulieren, dass das Tastverhältnis zwischen den Aktivitätsperioden (ON) und den Inaktivitätsperioden (OFF) konstant gehalten wird.

4. Vorrichtung nach Anspruch 1, wobei die Mittel zur dynamischen Steuerung der VNS Therapie Folgendes umfassen:
- Mittel, die dazu geeignet sind, in jedem Herzzyklus, die Überschreitung des Grenzwerts (S) durch den aktuellen Wert des Kontrollparameters (RR) zu überwachen; und
- Mittel, die dazu geeignet sind, in jedem Herzzyklus bei Überschreitung dieses Grenzwerts die Aktivitätsperiode zu beenden.

5. Vorrichtung nach Anspruch 1, wobei der Grenzwert (S) ein fester vorbestimmter Grenzwert ist.

6. Vorrichtung nach Anspruch 1, wobei der Grenzwert (S) ein dynamischer Grenzwert ist, und die Vorrichtung ferner Mittel zur Berechnung eines Grenzwerts des Kontrollparameters für jede aktuelle Aktivitätsperiode aufweist.

7. Vorrichtung nach Anspruch 6, wobei die Mittel zur Berechnung des Grenzwerts Mittel zur Berechnung des Grenzwerts in Abhängigkeit eines nach der Auslösung der aktuellen Aktivitätsperiode erreichten Extremwerts (P) des Kontrollparameters umfassen.

8. Vorrichtung nach Anspruch 7, wobei die Mittel zur Berechnung des Grenzwerts dazu geeignet sind, den Grenzwert in Abhängigkeit des gemessenen Unterschieds zwischen i) einem Ausgangswert des Kontrollparameters vor der Auslösung der aktuellen Aktivitätsperiode und ii) dem nach der Auslösung dieser aktuellen Aktivitätsperiode erreichten Extremwert des Kontrollparameters zu berechnen.

9. Vorrichtung nach Anspruch 7, wobei die Mittel zur Berechnung des Grenzwerts dazu geeignet sind, den Grenzwert in Abhängigkeit eines Ausgangswerts des Kontrollparameters vor der Auslösung der aktuellen Aktivitätsperiode zu berechnen.

10. Vorrichtung nach Anspruch 9, wobei die Mittel zur Berechnung des Grenzwerts dazu geeignet sind, den Grenzwert in Abhängigkeit des nach der Auslösung dieser aktuellen Aktivitätsperiode erreichten Extremwerts des Kontrollparameters zu berechnen.

11. Vorrichtung nach Anspruch 1, weiter umfassend: Mittel zur Detektion des Auftretens von deletären Erscheinungen und Mittel, die dazu geeignet sind, die Auslösung einer Sequenz von VNS-Impulsen durch den Generator im Falle des Auftretens eines deletären Ereignisses zu hemmen.

12. Vorrichtung nach Anspruch 11, wobei die deletären Erscheinungen Erscheinungen aus der Gruppe umfassend Husten, ventrikuläre Extrasystolen, Apnoe sind.

13. Vorrichtung nach Anspruch 11, ferner umfassend: Zeitsteuerungsmittel, die dazu geeignet sind, nach Ablauf einer vorbestimmten Zeitdauer die Erzeugung von Sequenzen von VNS Impulsen bedingungslos zu beenden.

## Claims

1. An active implantable medical device for VNS neuronal therapy by stimulation of the vagus nerve or of one of its branches, the device comprising:
- a generator (10) capable of producing VNS stimulation pulse sequences generated continuously in succession during activity periods (ON) separated by intermediate inactivity periods (OFF) during which no stimulation is issued;
- analysis means (22) receiving an input signal, provided by a physiological sensor, representative of the cardiac activity and/or the hemodynamic status of the patient carrying the device, and outputting to the generator a control parameter of the current efficiency for the VNS therapy; and
- means for dynamic control (24) of the VNS therapy, capable of modulating the duration of activity periods based on the current value of the control parameter issued by the analysis means,
**characterized in that**:
• the means for dynamic control of the VNS therapy are capable of modulating the duration of the activity period (ON) for each VNS stimulation cycle,
• and are further adapted to calculate, at the end of each activity period (ON), the duration of the inactivity period (OFF) depending on the duration of the preceding activity period (ON),
and **in that** the means for dynamic control of the VNS therapy comprise:
- means that are capable of monitoring the crossing of a threshold value (S) by the current value of the control parameter (RR); and
- means that are capable of ending the activity period when the threshold value is crossed.

2. The device according to claim 1, wherein the means for dynamic control of the VNS therapy are capable of modulating the duration of the activity periods (ON) by comparison of the control parameter with a predetermined threshold.

3. The device according to claim 1, wherein the means for dynamic control of the VNS therapy are capable of modulating the duration of the inactivity periods (OFF) so as to maintain a constant duty cycle ratio between activity periods (ON) and inactivity periods (OFF).

4. The device according to claim 1, wherein the means for dynamic control of the VNS therapy comprise:
- means that are capable of monitoring for each cardiac cycle the crossing of said threshold value (S) by the current value of the control parameter (RR); and
- means that are capable of ending, at each cardiac cycle, the activity period if the threshold value is crossed.

5. The device according to claim 1, wherein the threshold (S) is a fixed predetermined threshold.

6. The device according to claim 1, wherein the threshold (S) is a dynamic threshold and the device further comprises means for calculating a threshold of the control parameter for each current activity period.

7. The device according to claim 6, wherein the means for calculating the threshold comprise means for calculating the threshold based on an extremum value (P) of the control parameter achieved consecutively to the triggering of the current activity period.

8. The device according to claim 7, wherein the means for calculating the threshold are capable of calculating the threshold based on the measured difference between i) a base value of the control parameter before the triggering of the current activity period and ii) the extremum value of the control parameter reached after the triggering of the current activity period.

9. The device according to claim 7, wherein the means for calculating the threshold are capable of calculating the threshold based on a base value of the control parameter before the triggering of the current activity period.

10. The device according to claim 9, wherein the means for calculating the threshold are capable of calculating the threshold based on the extremum value of the control parameter reached after triggering of the current activity period.

11. The device according to claim 1, further comprising means for detecting the occurrence of deleterious events, and means for inhibiting the triggering of a VNS stimulation pulse sequence by the generator in case of occurrence of a deleterious event.

12. The device according to claim 11, wherein the deleterious event is at least one of a cough, ventricular extrasystole, and apnea.

13. The device according to claim 11, further comprising means for timing control, adapted to unconditionally stop the generation of the VNS stimulation pulse sequence after lapse of a predetermined period.
